# EUROPEAN PATENT SPECIFICATION

(11) **EP 1 675 500 B1**
(45) Date of publication and mention of the grant of the patent: **15.05.2013**
(21) Application number: 04795399.7
(22) Date of filing: 15.10.2004
(51) Int. Cl.: A61B 17/34

(54) **EXPANDIBLE SURGICAL ACCESS DEVICE**
EXPANDIERBARE CHIRURGISCHE ZUGANGSVORRICHTUNG
DISPOSITIF D'ACCES CHIRURGICAL EXTENSIBLE

(30) Priority: 17.10.2003 US 512389 P
(43) Date of publication of application: 05.07.2006
(73) Proprietor: Covidien LP, Mansfield, MA 02048 (US)
(72) Inventor: SMITH, Robert, C., Cheshire, CT 06410 (US)
(74) Representative: Soames, Candida Jane
(86) International application number: PCT/US2004/034227
(87) International publication number: WO 2005/037079

(56) References cited:
- EP-A1- 0 542 428
- US-A- 5 330 501
- US-A- 5 445 615
- US-A- 5 445 615
- US-A- 5 618 309
- US-A- 5 618 309
- US-A- 5 755 697

## Description

### BACKGROUND

### 1. Technical Field

The present disclosure relates generally to surgical access devices, and, in particular, relates to an access device having an anchoring arrangement to securely engage the abdominal wall thereby minimizing the potential of inadvertent removal of the access device from the tissue site.

### 2. Background of Related Art

Minimally invasive surgical procedures including both endoscopic and laparoscopic procedures permit surgery to be performed on organs, tissues and vessels far removed from an opening within the tissue. These procedures typically employ a surgical instrument introduced into the body through a cannula which provides access to the underlying tissues within the tissue site. The cannula often incorporates a seal assembly adapted to provide a fluid tight seal about the instrument to minimize the leakage of insufflation gases from the body cavity.

While minimally invasive surgical procedures have proven to be quite effective in surgery, several disadvantages remain. The cannula may have a tendency to back out of the incision in the abdominal wall particularly during manipulation of the instruments through the cannula seal.
EP0542428 describes a surgical trocar assembly for insertion of a laparoscopic instrument through an inner abdominal wall. The preamble of appended claim 1 is based on its disclosure.
US5755697 discloses a device to assist percutaneous vein catheterization of patients.
US5330501 describes a tissue gripping device for use with a cannula.

### SUMMARY

The aspects and embodiments of the present invention are set forth in the accompanying claims.

The present disclosure relates to an access device for facilitating access to a surgical site. The access device includes an access member defining a longitudinal axis and having proximal and distal ends. The access member includes an inner member and an outer member disposed about the inner member. The inner member defines an opening therethrough to permit access to a surgical site. The inner member and outer member are moveable relative with respect to one another. The access device also includes a deployment member associated with the inner member and the outer member. The deployment member is adapted to be deployed in at least a radial outward direction relative to the longitudinal axis upon movement of the inner member and outer member relative to one another, to thereby be positioned to engage body tissue to facilitate retention of the access member within a patient's body. The deployment member comprises a deployment collar engaged with the inner member at a first end portion and the outer member at a second end portion of the collar.

The collar is desirably disposed adjacent a distal end of the inner member. In certain embodiments, the collar is disposed between the inner member and the outer member.

The deployment member preferably includes at least one deployment segment adapted to deflect in at least a radial outward direction relative to the longitudinal axis. The at least one deployment segment may include at least one hinge whereby the deployment segment pivots along the hinge to deflect in at least a radial outward direction. In certain embodiments, the at least one deployment segment comprises a plurality of bendable segments arranged so as to deflect in a radial outward direction upon movement of the inner member and the outer member with respect to each other in an axial direction.

In certain embodiments, the outer member defines an axial slot in an outer wall portion thereof, the at least one deployment segment being disposed inwardly of the outer member, and the axial slot permitting the bendable segment of the at least one deployment segment to pass therethrough upon deployment thereof.

The outer member desirably includes at least one thread portion on an exterior surface thereof, where the at least one thread portion is dimensioned for engaging tissue and cooperating with the deployment means so as to retain the apparatus in tissue. The at least one thread portion may comprise a plurality of thread portions arranged in interrupted manner about the exterior surface of the outer member.

In certain embodiments, the access apparatus further comprises a cam member in operative engagement with the inner member or the outer member. The cam member is moveable to drive the inner member or outer member in an axial direction to cause deployment of the at least one deployment segment. The cam member is desirably adapted for rotational movement. In certain embodiments, the cam member is in operative engagement with an inner housing attached to a proximal end of the inner member and an outer housing attached to a proximal end of the outer member, whereby rotational movement of the cam member causes movement of the inner member and the outer member with respect to one another in an axial direction and deployment of the at least one deployment segment. Desirably, at least one of the inner housing and the outer housing includes a cam slot and the cam member is in operative engagement with the cam slot to cause deployment of the deployment means. The cam member may be rotatably attached to the inner housing and the outer housing may have the cam slot so that rotation of the cam member advances the outer member in a distal direction.

In certain embodiments, the access apparatus includes a lever mechanism having a lever rotatably mounted to the inner member and in operative engagement with the outer member. The lever member is desirably rotatable to drive the outer member to cause deployment of the deployment means.

In certain embodiments, the access apparatus includes a rotatable control knob rotatably mounted to the inner member and in operative engagement with the outer member. The lever member is desirably rotatable to drive the outer member to cause deployment of the deployment means. The collar may be disposed outwardly from the inner member.

In certain embodiments, the deployment collar includes tabs and the outer member includes slots. The tabs are received in the slots. The apparatus may include a locking collar having a recess for engaging the distal end of the deployment collar. The locking collar is attached to the inner member.

In a further aspect of the present disclosure, an access apparatus for facilitating access to a surgical site comprises an access member defining a longitudinal axis and having an inner member and an outer member disposed about the inner member. The inner member defines an opening therethrough to permit access to a surgical site. The inner member and the outer member are movable with respect to one another in the axial direction. The apparatus includes a collar having a proximal end and a distal end, the proximal end being attached to the outer member and the distal end being attached to the inner member. The collar has deployment segments arranged to deflect in a radial outward direction upon movement of the inner member and outer member with respect to one another. A cam member is attached to one of the inner member and the outer member for engaging a surface on the other of the inner member and the outer member so that rotation of the inner member or the outer member moves the inner member and the outer member with respect to one another in the axial direction.

The inner member desirably has a groove for engaging the distal end of the collar. The deployment segments may have a proximal hinge and a distal hinge and may be arranged to bow outwardly at a central area of the deployment segments. The deployment segments may be arranged to bow outwardly at a central area of the deployment segments. The deployment segments may have a third hinge at the central area.

In certain embodiments, the collar is disposed inwardly of the outer member and the outer member defines axial slots. The deployment segments may extend through the axial slots after deployment.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
FIG. 1 is a perspective view of the apparatus in accordance with an embodiment of the present disclosure;
FIG. 1A is an enlarged perspective view of the distal end of the apparatus in accordance with the embodiment of FIG. 1;
FIG. 2 is another perspective view of the apparatus in accordance with the embodiment of FIGS. 1 and 1A;
FIG. 3 is a perspective view of the apparatus in accordance with the embodiment of FIGS. 1-2, showing a trocar mounted thereto;
FIG. 4 is an enlarged exploded view of the apparatus in accordance with the embodiment of FIGS. 1-3, showing parts separated illustrating the various components;
FIG. 5 is an enlarged perspective view of the deployment member of the apparatus in accordance with the embodiment of FIGS. 1-4;
FIG. 6 is an enlarged perspective view of the outer sheath of the apparatus in accordance with the embodiment of FIGS. 1-5;
FIG. 7 is a cross-sectional view of the apparatus in accordance with the embodiment of FIGS. 1-6;
FIG. 8 is an enlarged sectional view of the distal end of the apparatus in accordance with the embodiment of FIGS. 1-7;
FIG. 9 is a view in cross-section illustrating an apparatus in accordance with the embodiment of FIGS. 1-8, showing a trocar positioned within the apparatus and penetrating tissue;
FIG. 10 is a cross-sectional view in accordance with the embodiment of FIGS. 1-9, illustrating the deployment member in a deployed condition;
FIG. 11 is an enlarged isolated cross-sectional view of the distal end of the apparatus in accordance with the embodiment of FIGS. 1-10, further illustrating the relationship of the deployment member with the inner and outer sheath of the apparatus;
FIG. 12 is a perspective view of the apparatus in accordance with the embodiment of FIGS. 1-11, further illustrating the deployment member in a deployed condition; and
FIG. 13 is a cross-sectional view of the apparatus in accordance with the embodiment of FIGS. 1-12, showing the trocar removed to permit access to the underlying body cavity.
FIG. 14 is a perspective view of an apparatus which is not in accordance with the present invention ;
FIG. 15 is cross-sectional view of the apparatus taken along the lines 15-15 of FIG. 14;
FIG. 16 is an exploded view of the apparatus in accordance with the embodiment of FIGS. 14-15, with parts separated;
FIG. 16A is an enlarged isolated view of the locking groove of the inner sheath in accordance with the embodiment of FIGS. 14-16;
FIG. 16B is an enlarged isolated view illustrating the locking tabs of the deployment member in accordance with the embodiment of FIGS. 14-16A;
FIG. 17 is a cross-sectional view of the apparatus in accordance with the embodiment of FIGS. 14-16B;
FIG. 17A is an enlarged sectional view of the apparatus in accordance with the embodiment of FIGS. 14-17, illustrating the relationship of the cam member and the housing of the inner member;
FIG. 18 is a side perspective view of the base of the outer member of the apparatus in accordance with the embodiment of FIGS. 14-17A;
FIG. 18A is an enlarged view illustrating further details of the base in accordance with the embodiment of FIGS. 14-18;
FIG. 19 is a perspective view of the cam member in accordance with the embodiment of FIGS. 14-18A;
FIG. 19A is an enlarged view illustrating further details of the cam member in accordance with the embodiment of FIGS. 14-19;
FIG. 20 is a perspective view of the proximal end of the apparatus in accordance with the embodiment of FIGS. 14-19A;
FIG. 21 is a cross-sectional view of the apparatus taken along lines 21-21 of FIG. 20;
FIG. 22 is a perspective view similar to the view of FIG. 20 prior to mounting of the outer member to the inner member;
FIG. 23 is an enlarged sectional view of the distal end of the apparatus in accordance with the embodiment of FIGS. 14-22;
FIG. 24 is a view similar to the view of FIG. 15 illustrating the cam member actuated to deploy the deployment member;
FIG. 25 is a side view of the apparatus in accordance with the embodiment of FIGS. 14-24, illustrating advancement of the outer member upon actuation of the cam member;
FIG. 26 is a cross-sectional view of the apparatus in accordance with the embodiment of FIGS. 14-25, illustrating the deployment member fully deployed;
FIG. 27 is a perspective view of the apparatus in accordance with the embodiment of FIGS. 14-26, further illustrating the deployment member in a deployed condition;
FIG. 28 is a cross-sectional view of the apparatus in accordance with the embodiment of FIGS. 14-27, showing the apparatus deployed within body tissue;
FIGS. 29-32 are views of another embodiment of the present disclosure; and
FIGS. 33-36 are views of yet another embodiment of the present disclosure.

### DETAILED DESCRIPTION OF PREFERRED EMBODIMENTS

Preferred embodiment(s) of the apparatus of the present disclosure will now be described in detail with reference to the drawings wherein like reference numerals identify similar or like elements throughout the several views. As used herein, the term "distal" refers to that portion which is further from the user, while the term "proximal" refers to that portion which is closest to the user.

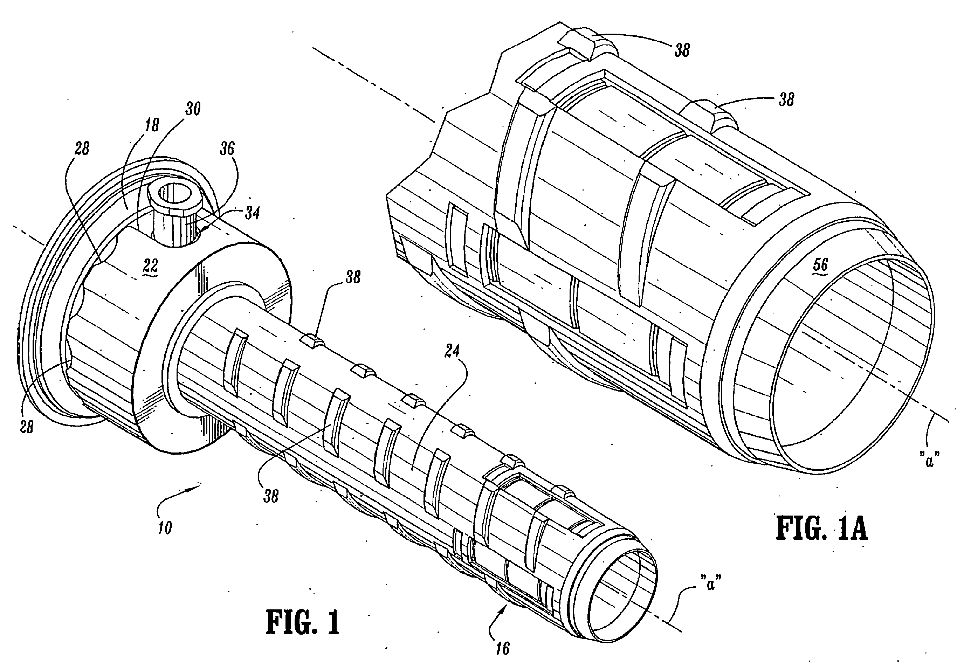

Referring initially to FIGS. 1-3, there is illustrated an apparatus in accordance with an embodiment of the present disclosure. Apparatus 10 is intended to permit access to body tissue, particularly, a body cavity, to permit the introduction of an object therethrough for performing various surgical procedures on internal organs within the cavity. The object may be a surgical instrument such as a laparoscopic or endoscopic clip applier, stapler, forceps, dissector, retractor, electro-surgical device or the like. Alternatively, the object may be the surgeon's arm or hand, e.g., when used during procedures where the hand is introduced within the body, such as the abdominal cavity, to directly assist in the required surgery. Apparatus 10 includes a mechanism which upon deployment secures the apparatus within the body tissue thereby minimizing the potential of the apparatus being unintentionally dislodged from its location.

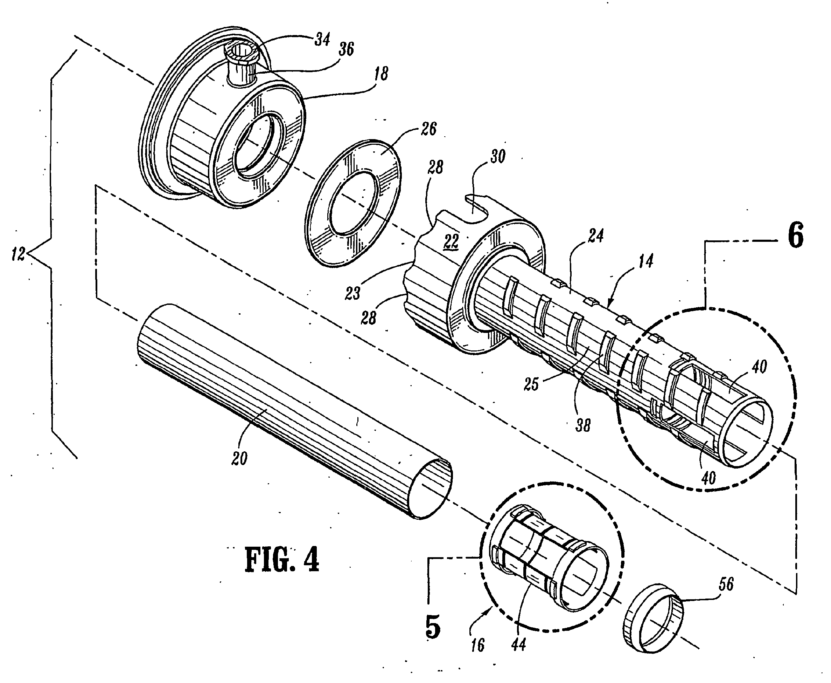

With reference to FIG. 4, in conjunction with FIGS 1-3, apparatus 10 generally includes three components, namely, first or inner member 12, second or outer member 14 coaxially mounted relative to inner member 12 about longitudinal axis "a" and deployment member 16 adjacent the distal ends of inner and outer members 12, 14. Inner member 12 includes housing 18 and inner sheath 20 extending distally from the housing 18. Similarly, outer member 14 includes base 22 and outer sheath 24 extending from the base 22. In the assembled condition of apparatus 10, inner member 12 is disposed within outer member 14 with housing 18 residing within base 22 and inner sheath 20 positioned within outer sheath 24. An elastomeric seal 26 may be positioned within base 22 to seal the interface between the base 22 and housing 18. Inner member 12 is axially movable relative to outer member 14 to deploy deployment member 16 as will be appreciated from the description provided hereinbelow.

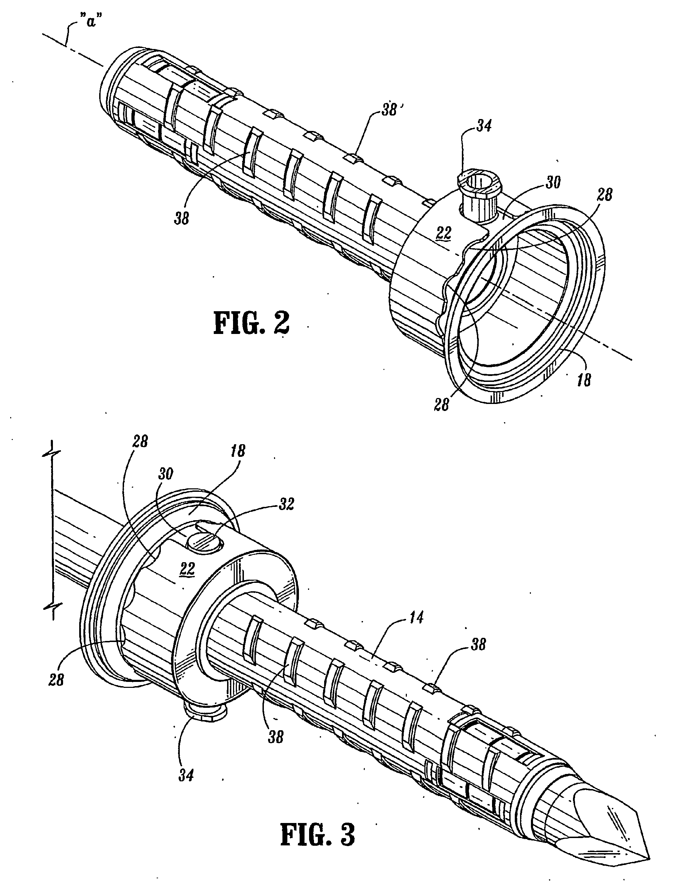

Base 22 of outer member 14 has a scalloped arrangement on its proximal surface 23 defined by a series of undulations or interconnected locking recesses 28. Base 22 further includes a pair of diametrically opposed enlarged indentations 30 also within its proximal surface. Housing 18 of inner member 12 includes boss 32 (FIG. 3) extending radially outwardly from its outer surface. Boss 32 is adapted to be received within one of locking recesses 28 of base 22 to lock deployment member 16 in the deployed position. Housing 18 further includes insufflation port 34 in diametrical opposed relation to boss 32. Insufflation port 34 defines a neck 36 which is also correspondingly dimensioned to be received in one of locking recesses 28 to facilitate retention of deployment member 16 in the deployed position. Insufflation port 34 permits passage of fluids into the body, such as insufflation of the abdominal cavity.

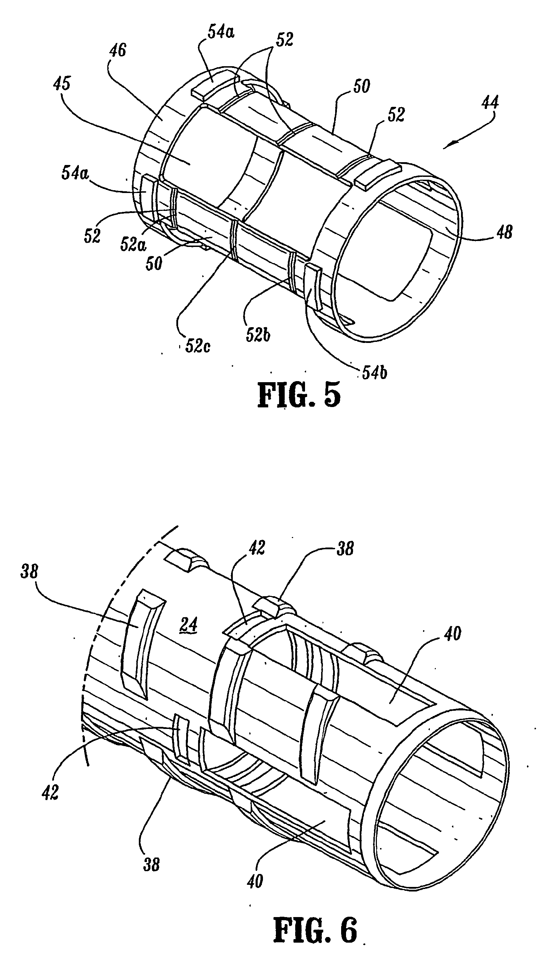

Inner sheath 20 of inner member 12 is a tube-like element and is secured to housing 18 by conventional means including adhesives, cements, welding and any other method known in the art. Alternatively, inner sheath 20 and housing 18 may be monolithically formed as a single unit. Outer sheath 24 is also tube-like and is mounted to permit rotational and axial movement of the outer sheath 24 relative to base 22. Outer sheath 24 defines an interrupted threaded configuration on the outer surface 23 of the outer sheath 25, having a series of partial threads 38, which assist in advancing the apparatus 10 within the surgical site through rotational movement, or in retaining the apparatus in tissue. As best depicted in FIG. 6, outer sheath 24 also includes a plurality of longitudinally extending axial slots 40 adjacent the distal end of the sheath 24 extending through the sheath 24 in a longitudinal direction of the outer sheath 24. A corresponding locking slot 42 is formed in outer sheath 24 adjacent each axial slot 40, proximal of the axial slots 40. The axial slots 40 and locking slots 42 cooperate to mount deployment member 16 and permit the deployment member 16 to assume the deployed position.

The threads formed in the outer sheath may comprise one or more continuous threads, or interrupted threads on the outer sheath 24. In further embodiments, the threads comprise one or more protrusions formed on the outer sheath, or may be omitted.

The components of inner member 12 and outer member 14 may be formed from any suitable rigid biocompatible material including, e.g., stainless steel, titanium, aluminum or a polymeric material including acrylics, styrene, carbonates and polymers thereof. Any suitable medical grade material may be used. Inner member 12 and outer member 14 may be opaque or transparent in whole or in part.

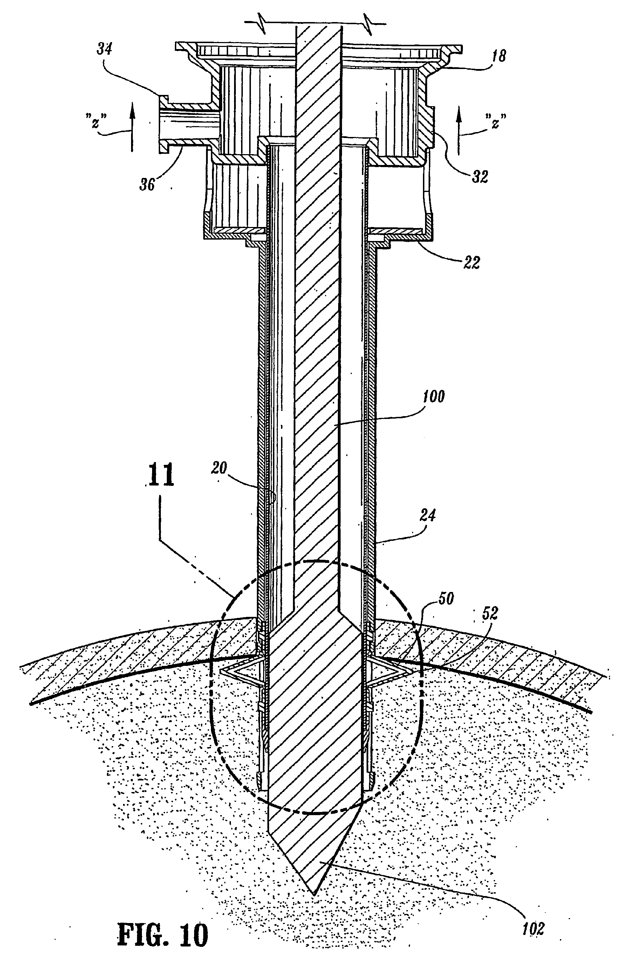

Referring now to FIGS. 1A and 4-6, deployment member 16 will be discussed. Deployment member 16 is part of a deployment means which secures apparatus 10 within the tissue site. Deployment member 16 includes deployment collar 44 which is shown in perspective view in FIG. 5. Deployment collar 44 includes first and second rings 46, 48 at respective proximal and distal ends of the collar 44 and interconnected by a plurality of deployment segments or tabs 50. Deployment segments 50 are radially spaced about collar 44 and define openings 45. Although four segments 50 are shown, the number of segments 50 may be greater or less than four. Deployment segments 50 each define a plurality of axially spaced hinge lines or joints 52. A first pair of hinge joints 52a, 52b is disposed adjacent respective first and second rings 46, 48 and a third hinge joint 52c is disposed at the approximate midpoint of deployment segment 50. Deployment segments 50 flex along hinge joints 52 to a radial outward position upon actuation of deployment member 16 to secure the apparatus 10 within the tissue of the body cavity (See FIG. 10). In further embodiments, the third hinge joint 52c may be omitted. Deployment segments 50 may bow at the approximate mid-point, without third hinge joint 52c.

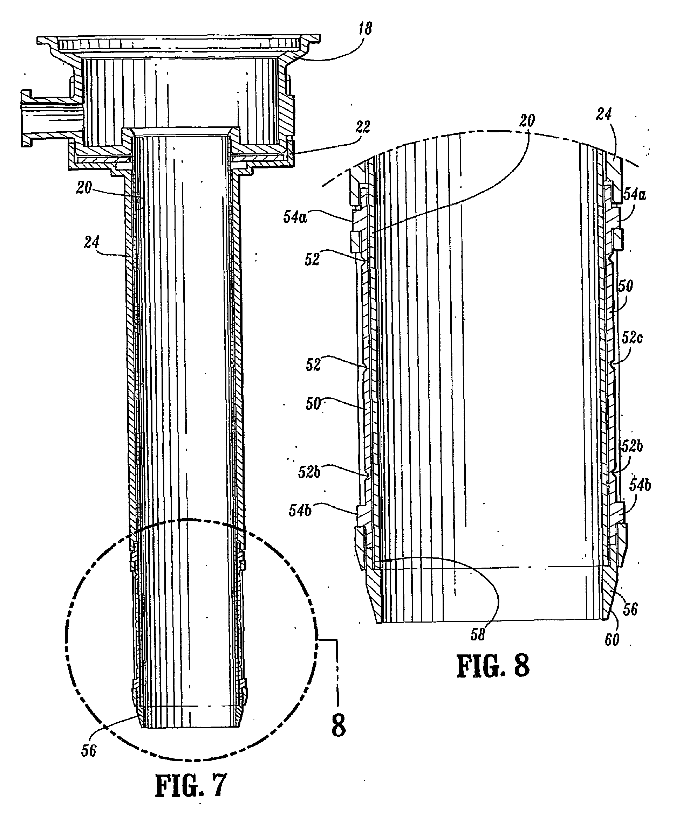

With continued reference to FIGS. 1A and 4-8, deployment collar 44 is mounted to inner and outer sheaths 20, 24 in the following manner. First and second rings 46, 48 each include a plurality of proximal and distal tabs 54 equi-distantly disposed about their respective peripheries and extending outwardly from the rings 46, 48. Proximal tabs 54a are correspondingly dimensioned and arranged to be received within locking slots 42 of outer sheath 24 to secure first ring 46 with respect to the outer sheath 24. With this arrangement, deployment segments 50 are aligned with axial slots 40 of outer sheath 24 thereby permitting the deployment segments 50 to bow outwardly through the axial slots 40 during deployment. In one embodiment, inner sheath 20 includes locking collar 56 mounted at its distal end. Locking collar 56 preferably includes an internal annular recess 58 which accommodates the distal end of inner sheath 20 as depicted in FIG. 8. Inner sheath 20 is secured to locking collar 56 adjacent internal annular recess 58 by conventional means including the use of adhesives, welding, or any other methods known in the art. Locking collar 56 further defines beveled end 60 which extends from the distal end of outer sheath. Beveled end 60 facilitates insertion of apparatus 10 within tissue. The proximal end of locking collar 56 abuts second ring 48 of deployment collar 44. Thus, as inner sheath 20 is retracted relative to outer sheath 24, locking collar 56 forces second ring 48 in a corresponding proximal or retracting direction. Such movement causes deployment segments 50 to flex outwardly along hinge joints 52 to a deployed condition. Distal tabs 54b ride within axial slots 40 of outer sheath 24 during the retracting movement.

In further embodiments, the deployment means comprises an inflatable membrane, or expandable sponge on the inner sheath, outer sheath or a collar associated with the inner and/or outer sheath. In further embodiments, the deployment segments 50 may be provided on outer sheath 24. In further embodiments, the deployment means is integrally formed with the inner sheath 20.

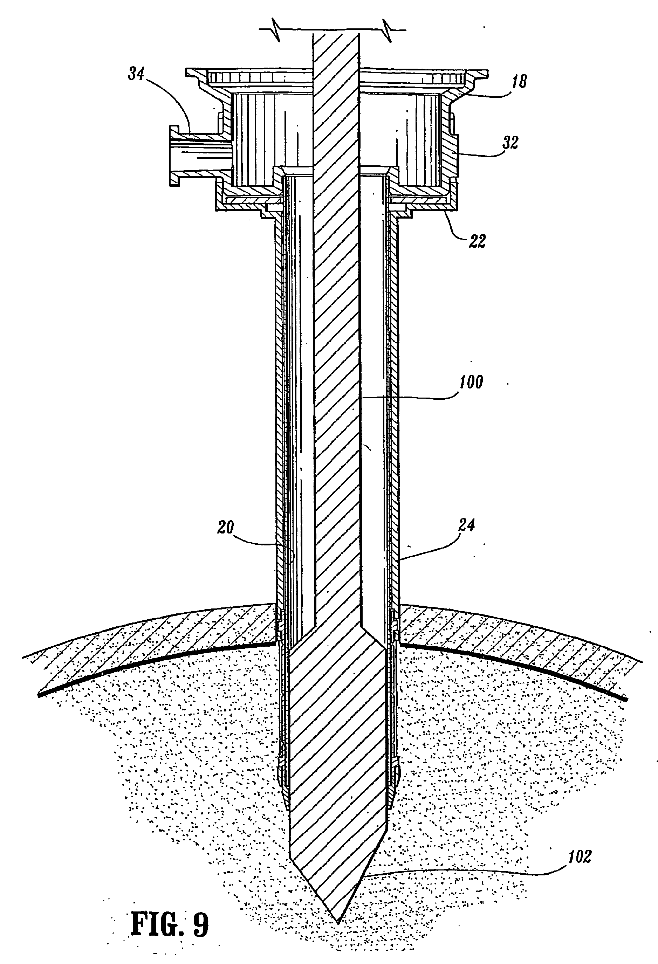

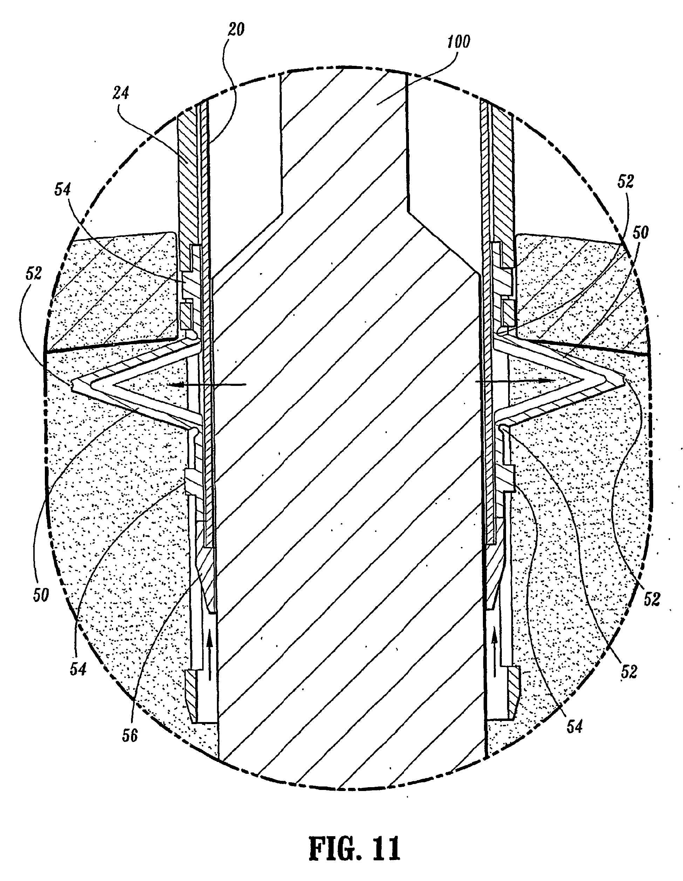

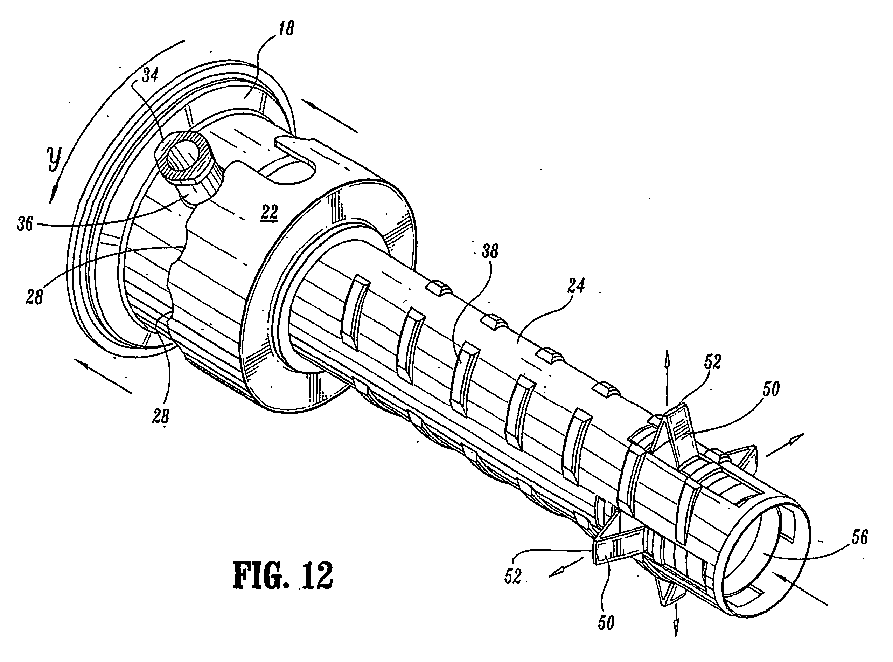

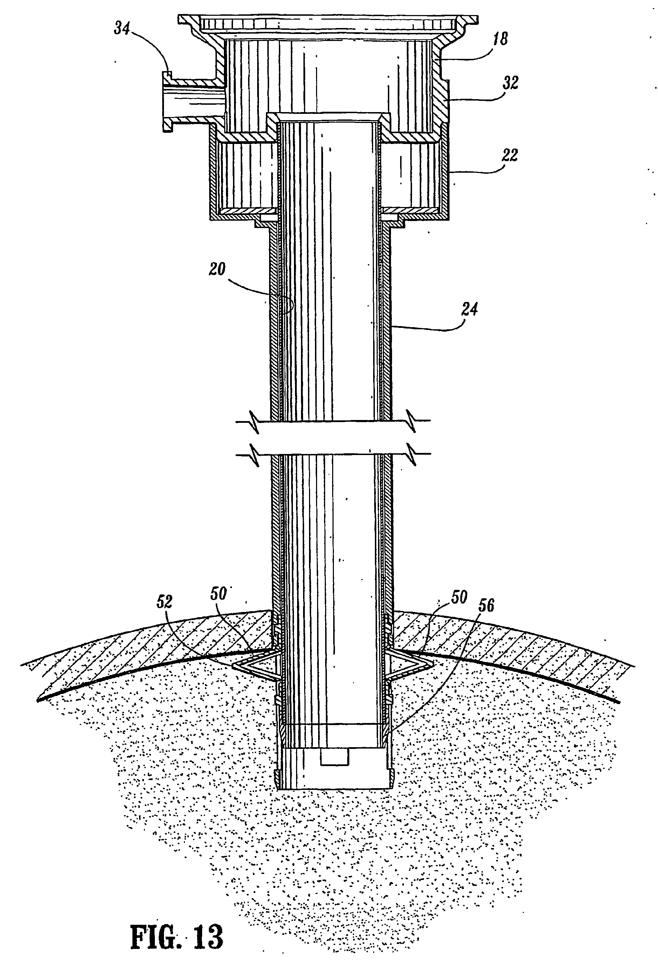

The operation of apparatus 10 will now be discussed. In a laparoscopic surgery, the peritoneal cavity is insufflated to raise the cavity wall to provide greater access to the tissue and organs within. With reference to FIG. 9, a trocar 100 is placed within apparatus 10 and advanced to extend the distal penetrating tip 102 into the tissue. The trocar 100 is used to puncture the abdominal wall as is conventional in the art. The trocar may then be removed if desired. Thereafter, inner member 12 is moved relative to outer member 14 in a proximal or retracted direction in the direction of directional arrows "z" as depicted in FIG. 10. Proximal movement of inner sheath 20 moves locking collar 56 proximally. Proximal movement of locking collar 56 causes second ring 48 of deployment collar 44, which is seated on the locking collar 56, to move proximally. This movement causes deployment segments 50 to bow outwardly along hinges 52 and extend through axial slots 40 of outer sheath 24 to the arrangement shown in FIG. 11. In this position, deployment segments 50 engage the inner wall of the peritoneal cavity thus preventing the apparatus from inadvertent withdrawal from the operative site. Alternatively, deployment segments 50 may be manipulated to engage the tissue surrounding the opening, preventing apparatus 10 from being pulled out of the abdomen. Desirably, a collar is frictionally or otherwise engaged with outer sheath and is used to engage the outer surface of the abdominal wall, and cooperating with deployment member 16 to fix apparatus 10 in position. FIG. 12 illustrates deployment collar 44 in the fully deployed position. Apparatus 10 is then secured in the deployed position by rotating housing 18 of inner member 12 in the direction of directional arrow "y" of FIG. 12. Inner sheath 20 and locking collar 56 rotate together with respect to deployment collar 44, as deployment collar 44 is not attached to locking collar 56. Alternatively, the inner sheath 20 is engaged with housing 18 so as to allow such rotation. Boss 32 and neck 36 of insufflation port 34 are then positioned in respective recesses 28 of base 22 of outer member 14. The boss 32 may be omitted in other embodiments. In this position, inner member 12 is secured in the retracted position thereby maintaining deployment collar 44 in the deployed condition. FIG. 13 illustrates apparatus with deployment member fully deployed and trocar removed to permit access to internal organs within the body cavity. Thereafter, an object such as a surgical instrument is introduced within the apparatus to perform the desired surgery. In further embodiments, the apparatus 10 is sized to receive a surgeon's hand, which is inserted into the body cavity.

It is contemplated that apparatus 10 may have a valve or seal assembly which may be mountable to housing 18, or incorporated into housing 18 and/or base 22. The preferred valve or seal assembly may include at least one valve or seal element adapted to form a seal about the inserted object to prevent release of insufflation gases through the apparatus 10. The valve or seal assembly may also include a zero-closure valve (e.g., a flapper or duck bill valve) to close the axial opening of the apparatus in the absence of the object. One valve assembly suitable for this purpose is disclosed in commonly assigned U.S. Patent No. 5,603,702 to Smith et al. . The '702 patent discloses, in certain embodiments, a valve assembly that may be adapted to mount to housing 18 through a detachable connection or the like including a bayonet coupling, friction fit, threaded connection or any other suitable connection known in the art. The valve assembly may be incorporated in the housing 18, base 22, or both.

In further embodiments, the trocar 100 is eliminated and a blunt obturator is used within the inner sheath 20. The apparatus 10 and obturator are advanced into the body after making an incision in the body tissue.

In certain embodiments, the deployment means is associated with the inner sheath and may comprise a portion of the inner sheath formed interally therewith or a separate collar mounted at a distal end of the inner sheath. The outer sheath has slots formed therein to accommodate the deployment of the deployment means, or the outer sheath is dimensioned to allow deployment of deployment means. The distal end of the inner sheath may be connected to the outer sheath so that upon distal movement of the inner sheath, the deployment segments extend through axial slots in outer sheath.

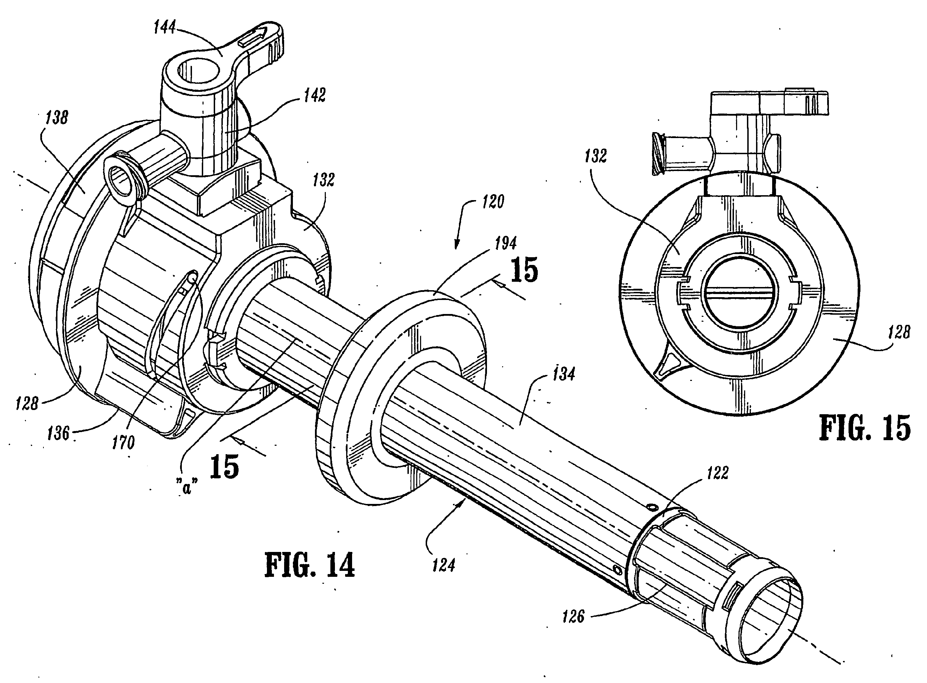

Referring to FIGS. 14-17, an apparatus not in accordance with the present invention is illustrated. Apparatus 120 includes first or inner member 122, second or outer member 124 coaxially mounted relative to inner member 122 about longitudinal axis "a," and deployment member 126 adjacent the distal end of inner and outer members 122, 124. Inner member 122 includes housing 128 and inner sheath 130 extending distally from the housing 128. Similarly, outer member 124 includes base 132 and outer sheath 134 extending from the base 132. In the assembled condition of apparatus 120, inner member 122 is disposed within outer member 124 with housing 128 residing within base 132 and inner sheath 130 positioned within outer sheath 134. Apparatus further includes cam member 136 which is positioned within base 132, between housing 128 and base 132. Cam member 136 forms part of a cam mechanism which acts to deploy deployment member 126. Generally, cam member 136 is rotatable to axially move inner member 122 and outer member 124 relative to each other to deploy deployment member 126. The details and operation of cam member 136 will be discussed in greater detail hereinbelow.

In the embodiment shown, outer sheath 134 does not include threads, like those shown in FIG. 1. However, in further embodiment, continuous or interrupted threads maybe included.

Apparatus 120 further includes an adapter 138 which is positioned adjacent the proximal end of inner member 122 and mounted on housing 128. Adapter 138 mounts an elastomeric duck bill or zero seal 140. Such seal 140 closes in the absence of an object inserted into apparatus 120 to prevent passage of insufflation gases through the apparatus. Various means for mounting adapter 138 are envisioned including, e.g., a bayonet coupling, a snap fit arrangement, threaded arrangement, adhesives etc.. Apparatus 120 further includes an insufflation port 142 attached to housing 128 for introduction of insufflation fluids necessary to insufflate the abdominal cavity. A valve 144 is incorporated into insufflation port 142 as is known in the art.

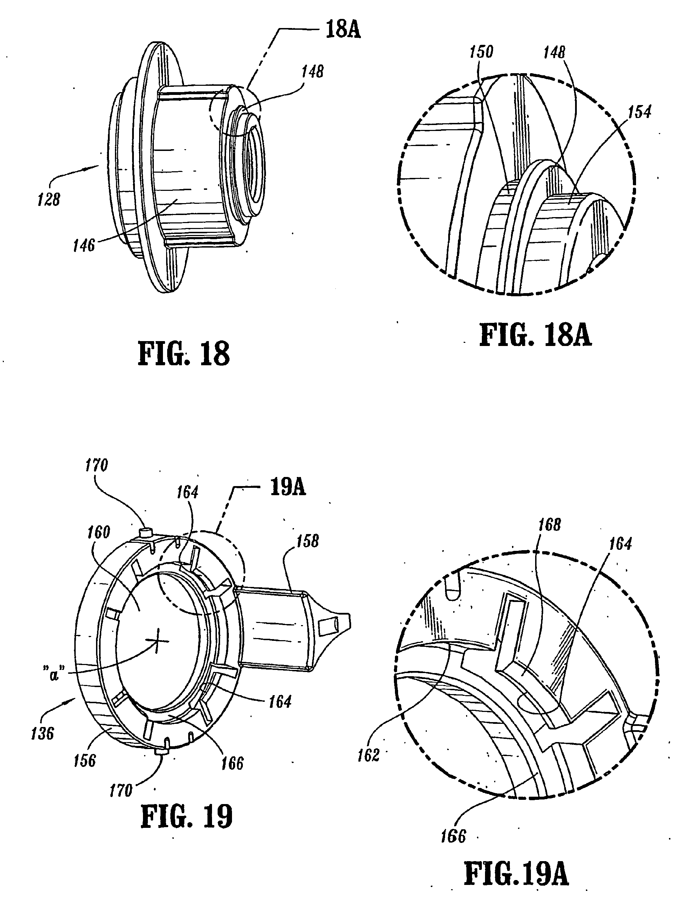

Referring to FIGS. 18, in conjunction with FIGS. 14-17, housing 128 of inner member 122 includes a recessed area or arc portion 146 in its outer surface and a circumferential rib 148 adjacent the distal portion of the housing 128. Circumferential rib 148 defines an annular or circumferential recess 150 which facilitates mounting of cam member 136 relative to housing 128. The proximal end of housing 128 has an enlarged flange 152. Flange 152 defines a support surface for supporting adapter 138. As appreciated, adapter 138 may be secured to flange 152 with the use of adhesives, etc.. if desired. The distal end of housing 128 includes a reduced diameter section 154.

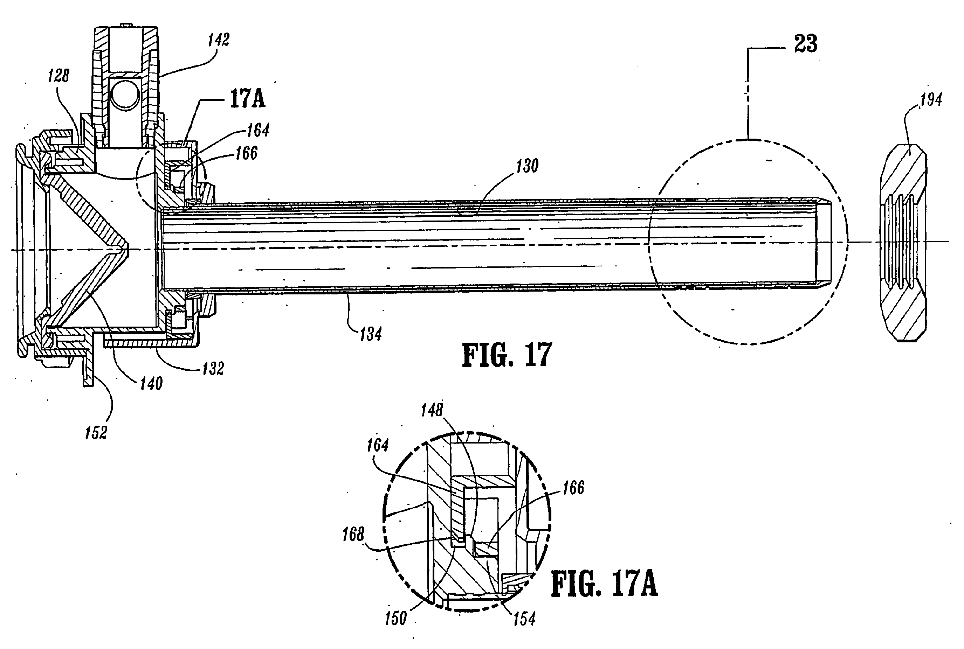

Referring now to FIGS. 19-22, in conjunction with FIGS. 17-18, cam member 136 will be described. Cam member 136 includes annular portion 156 and manually manipulative leg 158 extending from the annular portion 156. Annular portion 156 defines an opening 160 for coaxial mounting about reduced diameter section 154 of housing 128. Annular portion 156 further defines internal groove 162, a plurality of tabs 164 which extend radially inwardly adjacent the groove 162 and an internal rib 166 (FIGS. 19 and 19A). In the assembled position of cam member 136 on housing 128, tabs 164 are received within internal groove 150 of housing 128 with internal rib 166 of the cam member 136 in sliding contact relation with the reduced diameter portion 154 of housing 128. FIGS. 17 and 17A depict the relationship of these components in detail. Thus, as appreciated cam member 136 is free for rotational movement relative to housing 128, by virtue of the relationship of tabs 164 of cam member 136 and internal groove 150 of the housing 128, but is axially fixed to the inner member 122. Preferably, tabs 164 are sufficiently flexible to flex during assembly of the components to thereby permit the tabs 164 to flex to be received within internal groove 150 in general snap fit relation. Tabs 164 preferably include beveled surfaces 168 to facilitate this assembly process. Tabs 164 are arranged in spaced relation about annular portion 156.

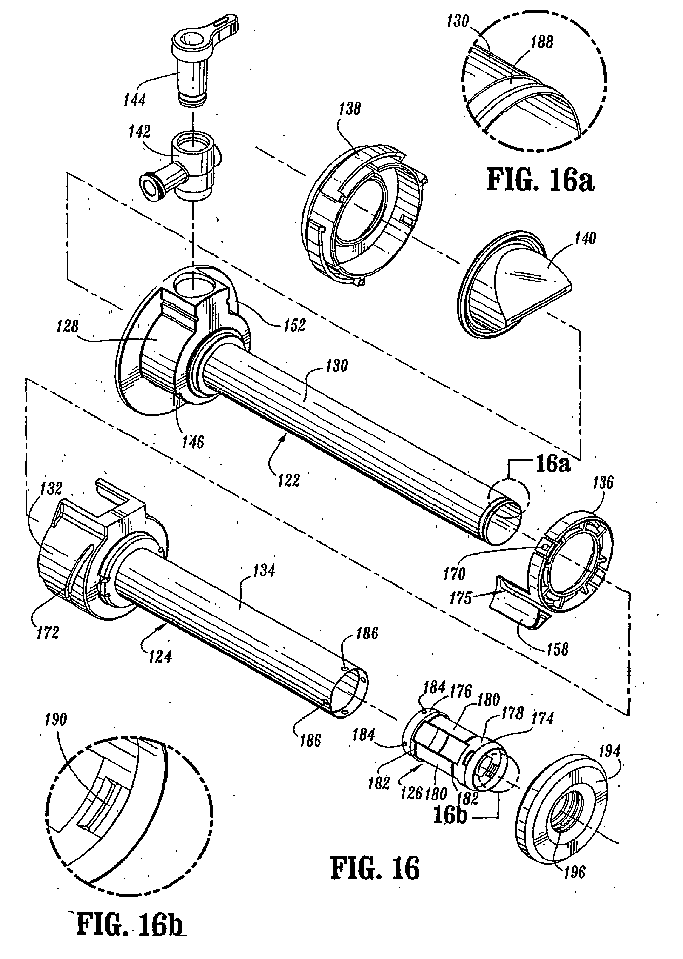

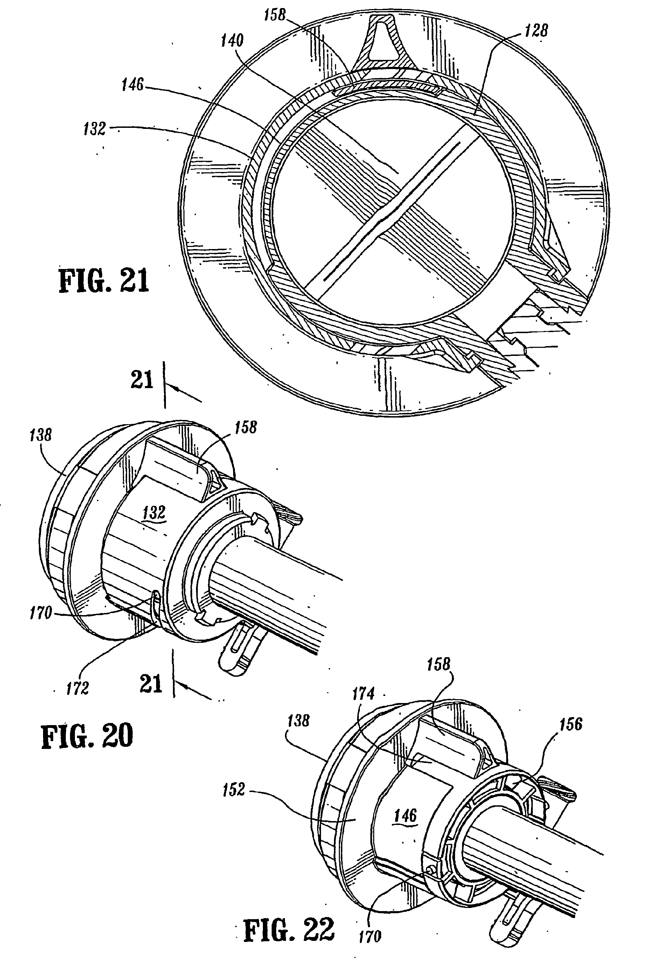

Annular portion 156 also includes first and second cam pins 170 arranged in general diametrical opposed relation and extending radially outwardly relative to the axis "a". Cam pins 170 are received within cam slots 172 defined within base 132 of outer member 124 and traverse the slots 172 during rotational movement of cam member 136 to drive movement of the outer member 124 and inner member 122 with respect to one another in the axial direction. Manually manipulative leg 158 of cam member 136 is accommodated within recessed arc portion 146 of housing 128 as best depicted in FIG. 21. Manually manipulative leg 158 further defines an axial slot 175 (FIGS. 16 and 22) dimensioned to receive a portion of the outer wall of base 132 when mounted to the base 132. FIG. 22 depicts cam member 136 mounted to housing 128 of inner member 122 prior to mounting of outer member 124. FIGS. 20 and 21 illustrate reception of the outer wall portion of base 132 within axial slot 174 of cam member 136 when outer member 124 is coaxially mounted about inner member 122.

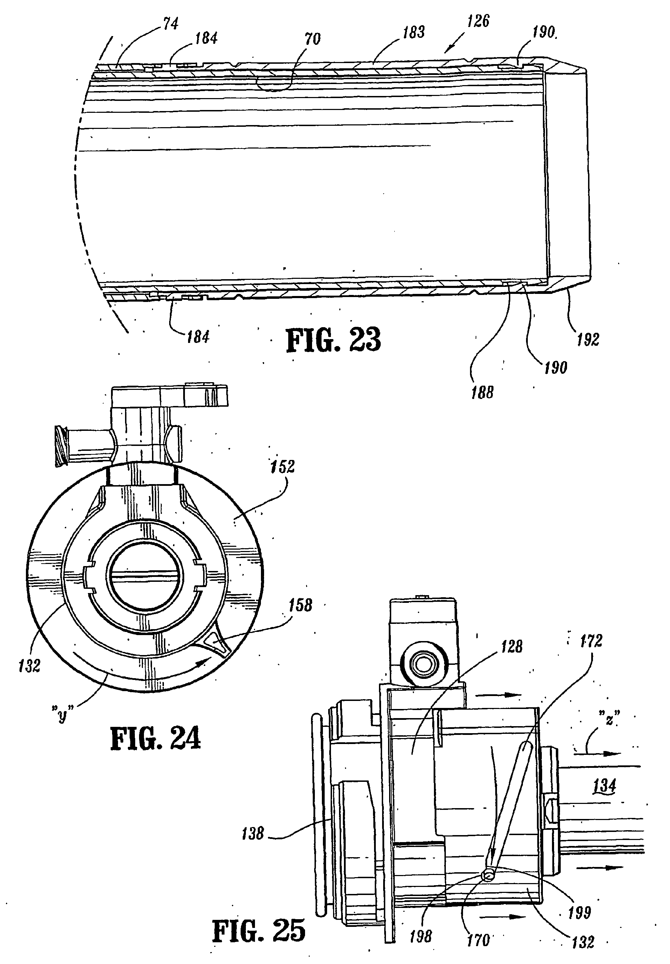

Referring now to FIG. 16 and 23, deployment member 126 will be discussed. Deployment member 126 is part of a deployment means which secures apparatus 120 within the tissue site. Deployment member 126 includes deployment collar 174, which is shown in perspective view in FIG. 16. Deployment collar 174 includes first and second rings 176, 178 at respective proximal and distal ends of the collar 174 and interconnected by a plurality of deployment segments 180 defining openings 181. Deployment segments 180 are radially spaced about collar 174. Although four segments 180 are shown, the number of segments 180 may be greater, e.g. five, six, or less than four. Deployment segment 180 each define a pair of axially spaced hinge lines or joints 182 disposed adjacent respective first and second rings 176,178. Deployment segments 180 flex along hinge joints 182 and bow outwardly adjacent its central area 183 to a radially outward position upon actuation of deployment member 126 to thereby secure apparatus 120 within the tissue of the body cavity.

Deployment collar 174 is mounted to inner and outer sheaths 130, 134 in the following manner. First ring 176 includes a plurality of pins 184 equi-distally disposed about its periphery and extending outwardly from the ring 176. Pins 184 are correspondingly dimensioned and arranged to be received within locking openings 186 of outer sheath 134 in snap fit relation to secure first ring 176 to the outer sheath 74. Second ring 178 includes a plurality of internal tabs 190 (FIG. 23) equi-distally disposed within the internal region of the ring 178. Tabs 190 are arranged to be received within groove 188 (FIG. 16A) of inner sheath 130 to secure second ring 178 to inner member 124. With this arrangement, deployment collar 174 is axially fixed to inner and outer sheath 130,134 thereby permitting the deployment segments 180 to bow outwardly during deployment. In one preferred embodiment, deployment collar 174 includes beveled end 192 at its distal end which extends from the distal end of second ring 178 (FIG. 23). Beveled end 192 facilitates insertion of apparatus 120 within tissue.

Referring again to FIGS. 14 and 16, apparatus 120 may further include a flexible slide ring or donut 194 coaxially mounted about outer sheath 74 of outer member 124. Donut 194 is adapted to traverse the outer sheath 124 to be positioned against the tissue of the body cavity to cooperate with deployment member 126 to further facilitate securement of the apparatus 120 within the tissue. Donut 194 is frictionally engaged with outer sheath 74 and may have internal ribs 196 to facilitate frictional engagement about outer sheath 74. Donut 194 preferably comprises an elastomeric material. In certain embodiments, the outer sheath includes threads on all or a portion of the outer surface of the outer sheath.

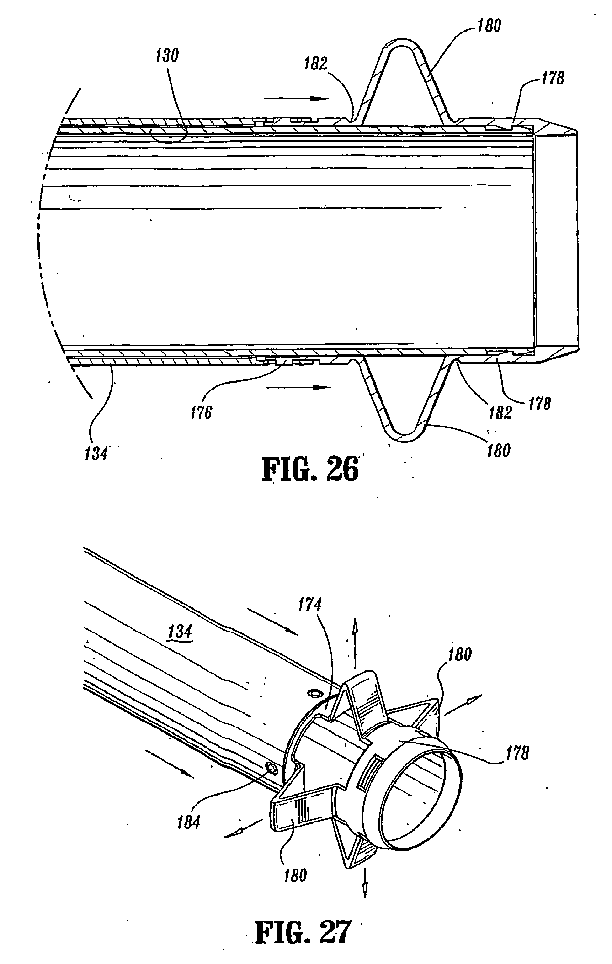

The operation of apparatus 120 will now be discussed. In a laparoscopic surgery, the peritoneal cavity is insufflated to raise the cavity wall to provide greater access to the tissue and organs within. A trocar is utilized to access the body cavity leaving apparatus 120 within the tissue site. With reference to FIG. 24, leg 158 of cam member 136 is moved in the direction of directional arrow "y". During this movement, cam pins 170 traverse cam slots 172 of base 132 as depicted in FIG. 25. The inclined or oblique relation of cam slots 172 causes outer member 124 (including base 132 and outer sheath 134) to be driven axially relative to inner member 122 in the distal direction, illustrated by directional arrows "z". As shown in FIG. 26, distal movement of outer sheath 134 causes first ring 176 of deployment collar 174 to move distally with respect to second ring 178. This movement causes deployment segments 180 to bow outwardly along hinge joints 182 to the arrangement shown in FIGS. 26 and 27. In this position, deployment segments 180 engage the inner wall of the peritoneal cavity preventing the apparatus from inadvertent withdrawal from the operative site. As shown in FIG. 26, deployment segments 180 define a bowed arrangement, as no hinge joint is located in the central area 183. The deployment segments 180 are desirably formed from a polymeric material, or other bendable material and sized so as to bend without a hinge joint in the central area 183. In further embodiments, the deployment segments 180 include a hinge joint in the central area 183, like joint 52c in FIG. 5. In further embodiments, the deployment segments are arranged to bend without any hinge joints. Desirably, deployment segments 180 have a curved shape in the bowed central area 183, whereas joint 52c creates a pointed shape in the central area of deployment segment 50. Alternatively, deployment segments 180 may be manipulated to engage the tissue surrounding the opening thus fixing the apparatus 120 within the incision.

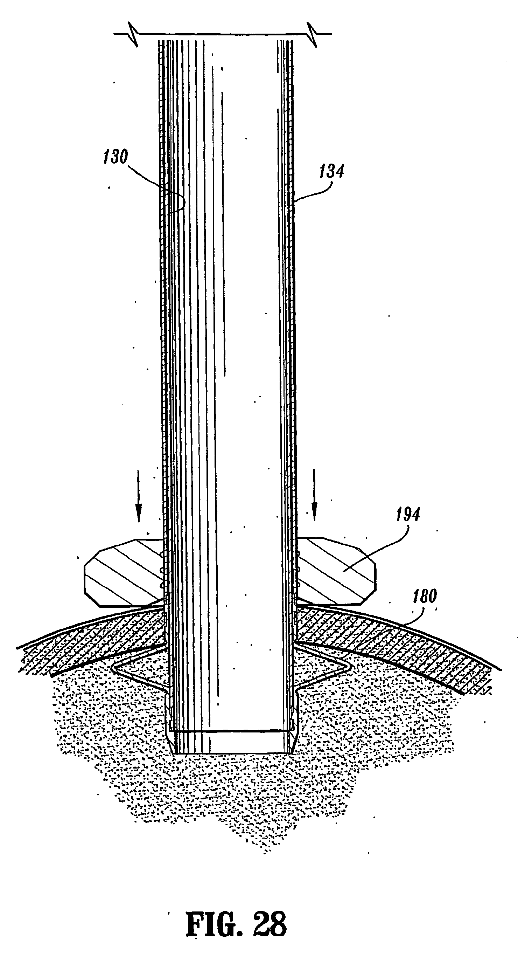

Cam member 136 may be secured in the deployed position by reception of cam pins 170 within recesses 198 of cam slots 172 (FIG. 24). As cam pins 170 are advanced, they override shelf 199 of base 132 adjacent cam slot 172. The shelf 199 defines the recess 198 of cam slot 172 so that the pin 170 is locked against the shelf 199 as shown in FIG. 25. It is envisioned that cam member 136 may be secured at a mid point position by provision of an additional shelf along the cam slot 172 in the central area of the slot 172. Thereafter, donut 194 is advanced along outer sheath 134 to engage the outer wall of the patient thus fixing the apparatus 120 within the incision. FIG. 28 illustrates apparatus 120 with deployment member 126 fully deployed with flexible donut 194 securely engaging the outer wall of the patient. Thereafter, an object such as a surgical instrument is introduced within the apparatus to perform the desired surgery. In further embodiments, the apparatus 120 is sized to receive a surgeon's hand, which is inserted into the body cavity.

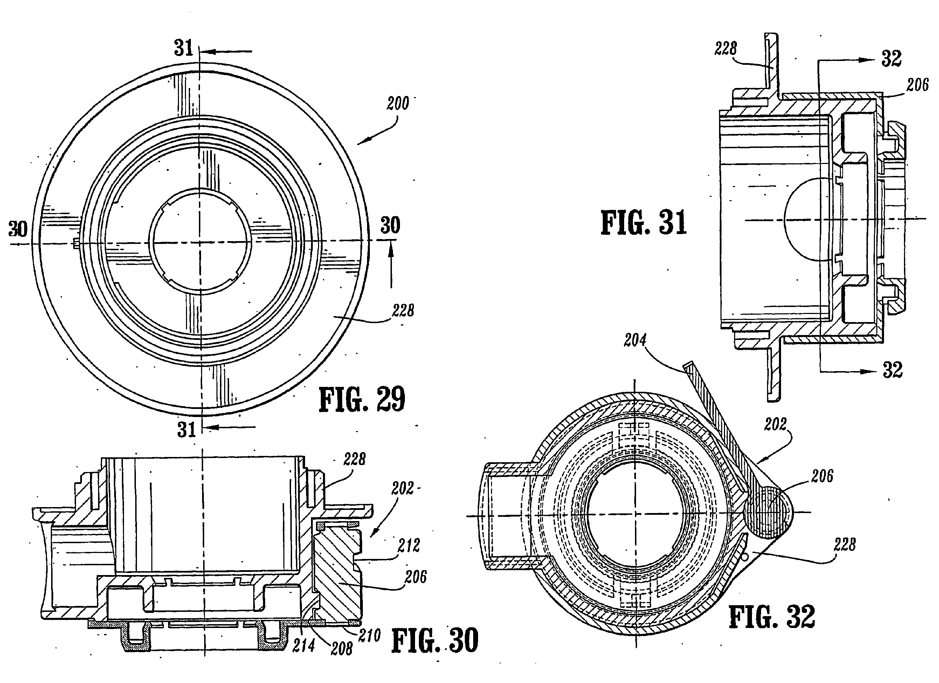

Referring now to FIGS. 29-32, there is illustrated another embodiment of the present disclosure. This device 200 is substantially similar to the device of FIGS. 14-28, and includes inner and outer members having associated inner and outer sheaths, a housing 228 and a base 232. However, with this embodiment, the cam mechanism is replaced with a lever mechanism 202 to drive outer member in the distal direction to deploy deployment member. Specifically, lever mechanism 202 includes manually engageable lever 204 which is mounted for rotational movement to housing 228 of inner member through lever pin 206. Lever pin 206 is engageable with collar 208 and is rotatably received within an aperture 210 in the collar 208. Collar 208, in turn, is connected to outer sheath through conventional means. Lever pin 206 includes a helical groove 212 in its outer periphery which receives a corresponding transverse groove pin 214 associated with housing 228. Consequently, rotation of lever 204 causes lever pin 206 to translate in the distal direction through traversing movement of helical groove 212 over groove pin 214. The distal movement of lever pin 206 causes collar 208 and outer member to be driven distally to deploy the deployment member in the aforedescribed manner described. The deployment member 16 shown in FIGS. 1-13, or deployment member 126 of FIGS. 14-28 may be used.

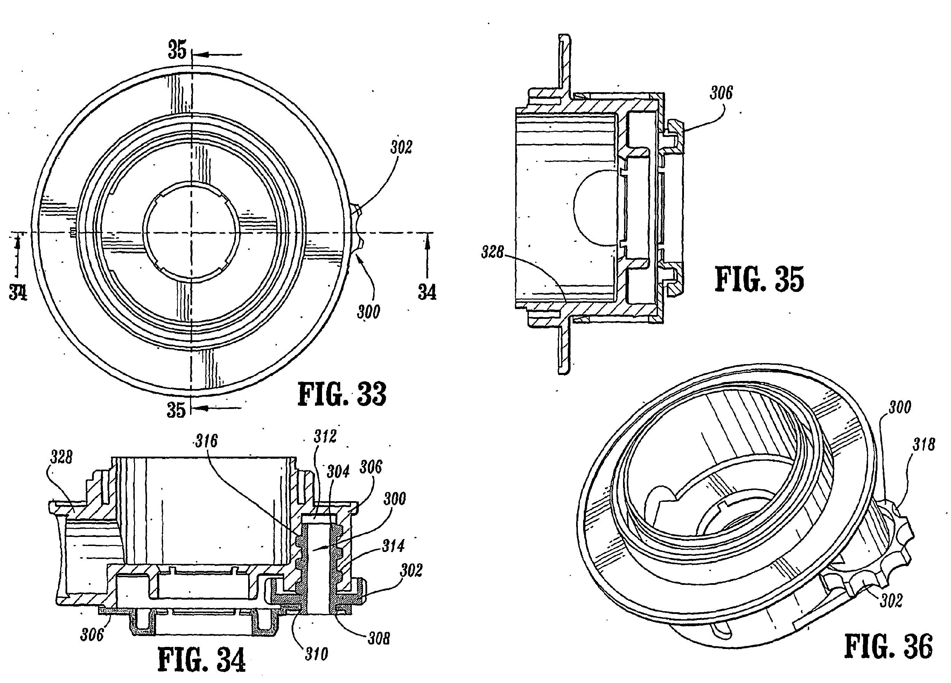

Referring now to FIGS. 33-36, there is illustrated another alternate embodiment of the present disclosure. This embodiment incorporates a rotatable control mechanism 300 to drive outer member to cause deployment of the deployment member. Rotatable control mechanism 300 includes rotatable knob 302 and pin 304 extending in the proximal direction from the knob 302. Knob 302 is operatively connected to collar 306 by reception of a depending portion 308 of the knob 302 within a corresponding opening 310 in the collar 306. Depending portion 308 is rotatable within opening 310. Pin 304 extends through an internal bore 312 associated with housing 328 of inner member 122. Pin 304 includes an external thread 314 which threadably engages internal thread 316 within the bore. Accordingly rotation of rotatable knob 302 causes corresponding rotation of pin 304 and thus corresponding axial movement of the knob 302 and pin 304 through the respective threaded arrangements to thereby drive the collar 306 and outer sheath to deploy the deployment member. Control knob 302 may include a scalloped outer surface 318 to facilitate engagement by the surgeon. It is noted that control knob 302 may be selectively rotated to cause partial deployment of the deployment member.

In the embodiments discussed above, the deployment member 16 or deployment member 126 carries a relatively thin elastomeric film on the deployment member to provide a seal with the tissue, to prevent the escape of insufflation gases during surgery.

It will be understood that various modifications may be made to the embodiments disclosed herein. Therefore, the above description should not be construed as limiting, but merely as exemplifications of preferred embodiments. Those skilled in the art will envision other modifications within the scope of the claims appended hereto.

## Claims

1. An access apparatus (10) for facilitating access to a surgical site, which comprises:
an access member defining a longitudinal axis and having proximal and distal ends, the access member including an inner member (12) including housing (18) and inner sheath (20) extending distally from the housing (18) and an outer member (14) including base (22) and outer sheath (24) extending from the base (22) and disposed about the inner member (12), the inner member (12) defining an opening therethrough to permit access to a surgical site, the inner member (12) and outer member (14) being moveable relative to each other and the outer sheath (24) including axial slots (40); and
a deployment member (16) associated with the inner member (12) and the outer member (14), the deployment member (16) comprising a deployment collar (44) and including at least one deployment segment (50) defining a plurality of axially spaced hinge joints (52) adapted to flex along hinge joints (52) to a radial outward position relative to the longitudinal axis upon movement of the inner member (12) and outer member (14) relative to one another, and **characterised in that**
the deployment collar (44) has a distal end engaged with the inner member (12)) and a proximal end engaged with the outer member (14) and is concentrically disposed between the inner member (12) and the outer member (14) and wherein said at least one deployment segment (50) of the deployment collar (44) is adapted to extend through axial slots (40) of said outer sheath (24) of the outer member (14) to thereby be positioned to engage body tissue to facilitate retention of the access member within a patient's body.

2. The access apparatus (10) of claim 1, wherein the deployment collar (44) is disposed adjacent a distal end of the inner member (12).

3. The access apparatus (10) according to claim 1, wherein the outer member (14) includes at least one thread portion (38) on an exterior surface thereof, the at least one thread portion dimensioned for engaging tissue and cooperating with the deployment means so as to retain the apparatus in tissue.

4. The access apparatus (10) according to claim 3, wherein the at least one thread portion comprises a plurality of thread portions arranged in interrupted manner about the exterior surface of the outer member (14).

5. The access apparatus (10) according to claim 1, further comprising a lever mechanism (202) the lever mechanism including a lever (204) rotatably mounted to the inner member (12) and in operative engagement with the outer member (14), the lever member rotatable to drive the outer member (14) to cause deployment of the deployment member (16).

6. The access apparatus (10) according to claim 1, further comprising a rotatable control knob (302) rotatably mounted to the inner member (12) and in operative engagement with the outer member (14), the control knob (302) rotatable to drive the outer member (14) to cause deployment of the deployment means.

7. The access apparatus according to claim 1, wherein the deployment collar (44) includes tabs (50), the tabs (50 being received within the slots (40).

8. The access apparatus (10) according to claim 7, further comprising a locking collar (56) having a recess for engaging the distal end of the deployment collar (44), the locking collar (56) being attached to the inner member (12).

## Patentansprüche

1. Zugangsvorrichtung (10) zum Erleichtern des Zugangs zu einer Operationsstelle, umfassend:
ein Zugangselement, das eine Längsachse definiert und ein proximales und ein distales Ende aufweist, wobei das Zugangselement ein inneres Element (12), das ein Gehäuse (18) und eine innere Hülle (20), die sich distal vom Gehäuse (18) erstreckt, beinhaltet, und ein äußeres Element (14) beinhaltet, das eine Basis (22) und eine äußere Hülle (24), die sich distal von der Basis (22) erstreckt, beinhaltet und um das innere Element (12) herum angeordnet ist, wobei das innere Element (12) eine Öffnung durch sich hindurch definiert, um den Zugang zu einer Operationsstelle zu gestatten, wobei das innere Element (12) und das äußere Element (14) zueinander beweglich sind und die äußere Hülle (24) axiale Schlitze (40) beinhaltet, und
ein Einsatzelement (16), das mit dem inneren Element (12) und dem äußeren Element (14) verbunden ist, wobei das Einsatzelement (16) eine Einsatzmanschette (44) umfasst und mindestens ein Einsatzsegment (50) beinhaltet, das mehrere axial beabstandete Gelenkverbindungen (52) bildet und das dafür eingerichtet ist, sich mit der Bewegung des inneren Elements (12) und des äußeren Elements (14) zueinander entlang den Gelenkverbindungen (52) zu einer radial auswärtigen Position in Bezug auf die Längsachse zu biegen, und **dadurch gekennzeichnet, dass**
die Einsatzmanschette (44) ein distales Ende aufweist, das in das innere Element (12) eingreift und ein proximales Ende, das in das äußere Element (14) eingreift, und konzentrisch zwischen dem inneren Element (12) und dem äußeren Element (14) angeordnet ist, wobei das mindestens eine Einsatzsegment (50) der Einsatzmanschette (44) dafür eingerichtet ist, sich durch axiale Schlitze (40) der äußeren Hülle (24) des äußeren Elements (14) zu erstrecken, um dadurch so positioniert zu werden, dass es in Körpergewebe eingreift, um das Festhalten des Zugangselements im Körper eines Patienten zu erleichtern.

2. Zugangsvorrichtung (10) nach Anspruch 1, wobei die Einsatzmanschette (44) an ein distales Ende des inneren Elements (12) angrenzend angeordnet ist.

3. Zugangsvorrichtung (10) nach Anspruch 1, wobei das äußere Element (14) mindestens einen Gewindeabschnitt (38) an einer seiner Außenflächen beinhaltet, wobei der mindestens eine Gewindeabschnitt derart bemessen ist, dass er in das Gewebe eingreift und mit den Einsatzmitteln zusammenwirkt, sodass die Vorrichtung im Gewebe festgehalten wird.

4. Zugangsvorrichtung (10) nach Anspruch 3, wobei der mindestens eine Gewindeabschnitt mehrere Gewindeabschnitte umfasst, die in unterbrochener Weise um die Außenfläche des äußeren Elements (14) herum angeordnet sind.

5. Zugangsvorrichtung (10) nach Anspruch 1, ferner einen Hebelmechanismus (202) umfassend, wobei der Hebelmechanismus einen Hebel (204) beinhaltet, der drehbar am inneren Element (12) montiert ist und in funktionsmäßigem Eingriff mit dem äußeren Element (14) steht, wobei das Hebelelement drehbar ist, um das äußere Element (14) anzutreiben, um den Einsatz des Einsatzelements (16) zu bewirken.

6. Zugangsvorrichtung (10) nach Anspruch 1, ferner einen drehbaren Bedienungsknopf (302) umfassend, der drehbar an dem inneren Element (12) montiert ist und in funktionsmäßigem Eingriff mit dem äußeren Element (14) steht, wobei der Bedienungsknopf (302) drehbar ist, um das äußere Element (14) anzutreiben, um den Einsatz der Einsatzmittel zu bewirken.

7. Zugangsvorrichtung (10) nach Anspruch 1, wobei die Einsatzmanschette (44) Streifen (50) beinhaltet, wobei die Streifen (50) in die Schlitze (40) aufgenommen sind.

8. Zugangsvorrichtung (10) nach Anspruch 7, ferner eine Verrieglungsmanschette (56) umfassend, die eine Vertiefung zum Eingriff in das distale Ende der Einsatzmanschette (44) aufweist, wobei die Verrieglungsmanschette (56) an dem inneren Element (12) befestigt ist.

## Revendications

1. Dispositif d'accès (10) pour faciliter l'accès à un site chirurgical, qui comprend :
un élément d'accès définissant un axe longitudinal et ayant des extrémités proximale et distale, l'élément d'accès incluant un élément intérieur (12) incluant un logement (18) et une gaine intérieure (20) s'étendant de façon distale à partir du logement (18) et un élément extérieur (14) incluant une base (22) et une gaine extérieure (24) s'étendant à partir de la base (22) et disposée autour de l'élément intérieur (12), l'élément intérieur (12) définissant une ouverture à travers cette dernière pour permettre l'accès à un site chirurgical, l'élément intérieur (12) et l'élément extérieur (14) étant mobiles l'un par rapport à l'autre et la gaine extérieure (24) incluant des fentes axiales (40) ; et
un élément de déploiement (16) associé à l'élément intérieur (12) et à l'élément extérieur (14), l'élément de déploiement (16) comprenant un collier de déploiement (44) et incluant au moins un segment de déploiement (50), définissant une pluralité d'articulations charnière (52) espacées de façon axiale, conçu pour fléchir le long des articulations charnière (52) vers une position extérieure radiale par rapport à l'axe longitudinal lors d'un mouvement de l'élément intérieur (12) et de l'élément extérieur (14) l'un par rapport à l'autre, et **caractérisé en ce que**
le collier de déploiement (44) a une extrémité distale en prise avec l'élément intérieur (12) et une extrémité proximale en prise avec l'élément extérieur (14) et est disposé de façon concentrique entre l'élément intérieur (12) et l'élément extérieur (14) et dans lequel ledit au moins un segment de déploiement (50) du collier de déploiement (44) est conçu pour s'étendre à travers des fentes axiales (40) de ladite gaine extérieure (24) de l'élément extérieur (14) pour être ainsi positionné pour mettre en prise un tissu corporel pour faciliter le maintien de l'élément d'accès à l'intérieur du corps d'un patient.

2. Dispositif d'accès (10) selon la revendication 1, dans lequel le collier de déploiement (44) est disposé adjacent à une extrémité distale de l'élément intérieur (12).

3. Dispositif d'accès (10) selon la revendication 1, dans lequel l'élément extérieur (14) inclut au moins une partie de filet (38) sur une surface extérieure de celui-ci, l'au moins une partie de filet étant dimensionnée pour mettre en prise un tissu et coopérer avec le moyen de déploiement afin de maintenir le dispositif dans le tissu.

4. Dispositif d'accès (10) selon la revendication 3, dans lequel l'au moins une partie de filet comprend une pluralité de parties de filet agencées de façon interrompue autour de la surface extérieure de l'élément extérieur (14).

5. Dispositif d'accès (10) selon la revendication 1, comprenant en outre un mécanisme de levier (202), le mécanisme de levier incluant un levier (204) monté en rotation sur l'élément intérieur (12) et en prise opérationnelle avec l'élément extérieur (14), l'élément de levier pouvant tourner pour entraîner l'élément extérieur (14) pour amener le déploiement de l'élément de déploiement (16).

6. Dispositif d'accès (10) selon la revendication 1, comprenant en outre un bouton de commande rotatif (302) monté en rotation sur l'élément intérieur (12) et en prise opérationnelle avec l'élément extérieur (14), le bouton de commande (302) pouvant tourner pour entraîner l'élément extérieur (14) pour amener le déploiement du moyen de déploiement.

7. Dispositif d'accès (10) selon la revendication 1, dans lequel le collier de déploiement (44) inclut des pattes (50), les pattes (50) étant reçues à l'intérieur des fentes (40).

8. Dispositif d'accès (10) selon la revendication 7, comprenant en outre un collier de verrouillage (56) ayant une partie en retrait pour mettre en prise l'extrémité distale du collier de déploiement (44), le collier de verrouillage (56) étant attaché à l'élément intérieur (12).
